# EUROPEAN PATENT SPECIFICATION

(11) **EP 3 618 739 B1**
(45) Date of publication and mention of the grant of the patent: **11.01.2023**
(21) Application number: 18727464.2
(22) Date of filing: 15.05.2018
(51) Int. Cl.: A61B 17/34, A61B 8/08, A61B 10/02, A61B 90/00

(54) **MEDICAL NEEDLE COMPRISING ECHOGENIC ENHANCEMENTS**
MEDIZINISCHE NADEL MIT ECHOGENEN VERBESSERUNGEN
AIGUILLE MÉDICALE COMPRENANT DES AMÉLIORATIONS ÉCHOGÈNES

(30) Priority: 23.05.2017 SE 1750637
(43) Date of publication of application: 11.03.2020
(73) Proprietor: In Front Medtech AB, 184 94 Åkersberga (SE)
(72) Inventor: LINDGREN, Mikael, 194 55 Upplands Väsby (SE); LINDGREN, Fredrik, 184 94 Akersberga (SE)
(74) Representative: Bjerkén Hynell KB
(86) International application number: PCT/SE2018/050498
(87) International publication number: WO 2018/217151

(56) References cited:
- US-A1- 2003 135 117
- US-A1- 2010 168 684
- US-A1- 2014 265 024
- US-A1- 2017 049 993

## Description

### Technical field

The present disclosure relates to a medical needle, and in particular a medical needle having an improved ultrasound reflection capability.

### Background

Medical devices for subcutaneous use are known in the medical field. For example, biopsy needles are used to capture and remove internal tissues while avoiding invasive surgery. When performing medical procedures, often targeted bodily areas are surrounded by blood vessels or internal organs which can cause difficulties with accurate percutaneous positioning of medical devices. Imaging methods can mitigate some of these difficulties by providing for simultaneous imaging of internal organs and medical devices. Ultrasound imaging is particularly suitable due to its lesser operation cost and increased portability in comparison to other imaging modalities such as X-ray and MRI. During ultrasound imaging procedures, a transducer emits ultrasound waves. A portion of the ultrasound waves reflect when encountering organs, tissues, and other items inside the body and then return to the transducer. The returned sound waves are then used to produce a visual representation of an internal cavity. This provides a real-time moving image of the internal organs and medical device which a physician can use to guide the medical device to the desired bodily area.

Problems exist with current uses of ultrasound imaging to place a medical device subcutaneously because the image obtained through ultrasound is not always clear. However, there are ways to increase image clarity. Echogenic enhancements in the form of a matrix of depressions (i.e. dimples) in the surface of a medical device can cause an altered or improved reflective response of ultrasound waves. When applied to a medical device, echogenic enhancements can cause the medical device to have greater ultrasound image clarity. This in turn can increase positioning accuracy of the medical device.

The published patent application US-2014/0265024A1 relates to echogenic surfaces with pressed-dimple formations. It is disclosed embodiments of methods and devices for providing enhanced echogenicity to medical devices. The method includes rolling an impression roller having a plurality of protrusions at an outer surface against a medical device. The protrusions create depressions in the surface of the medical device. A guide roller forms a ridge of the depression into the depression to create a lobe. The lobe forms a pocket between a bottom surface of the depression and the lobe.

Also the published patent application US-2010/0168684A1 relates to echogenic enhancement for a needle. A needle includes a surface with a plurality of first ultrasound reflecting depressions formed therein. The first depressions are distributed along at least a portion of a length of the needle separated from one another by intervening sections. Each of the first depressions is extending along a curve between first and second ends adjacent to corresponding ones of the intervening sections with troughs at which surfaces of each of the first depressions most closely approach a longitudinal axis of the needle being offset toward the first ends of each of the first depressions.

US-2017/0049993 relates to echogenic enhancements to a medical needle providing enhanced visibility when the needle is inserted at a steep angle relative to the ultrasound transducer.

In the publication "Visibility of Ultrasound-guided Echogenic Needle and Its Potential in Clinical Delivery of Regional Anesthesia" (Tokai J Exp Clin Med., Vol. 39, No. 2, pp. 80-86, 2014) different needles used in ultrasound-guided regional anesthesia are discussed. One needle is provided with indentations of a so-called corner cube reflector (CCR) type. As the name indicates an indentation of that type has a shape of a corner of a cube.

Thus, various types indentations are suggested in the prior art in order to improve the ultrasound visibility.

The object of the present invention is to achieve an improved medical needle with regard to ultrasound visibility, and in particular the ultrasound visibility at angles nearly parallel to the longitudinal axis of the needle.

### Summary

The above-mentioned object is achieved by the present invention according to the independent claim.

Preferred embodiments are set forth in the dependent claims.

Thus, according an aspect of the invention a medical needle is provided that comprises a longitudinal body extending between a distal end and a proximal end along a longitudinal axis A. An outer surface of the needle includes a plurality of concave echogenic ultrasonic reflector depressions formed along at least a portion of a length of the needle to enhance the visibility of the needle under ultrasound guidance by scattering and reflecting back toward a transducer sound waves incident thereon. A row of the depressions are evenly distributed around the entire circumference of at least one circular cylindrical section of the needle, said circular cylindrical section having depressions around the entire circumference is a depression section. Each of the depressions comprises two non-planar reflector surfaces, a first reflector surface and a second reflector surface, wherein the first reflector surface is a semi-spherical surface, or essentially a semi-spherical surface, and the second reflector surface is a part of a cylindrical or a conical surface, wherein the second reflector surface having a longitudinal symmetry axis S that is inclined in the direction of the proximal end of the needle in relation to the longitudinal axis A of the needle by an inclination angle α being in the interval of 20 - 35 degrees.

It has been shown that due to the defined combination, and the features of the first and second reflector surfaces, a medical needle having improved reflective capabilities is provided.

According to one embodiment the first surface of the depression ranges from a steep portion extending from a first end portion of the depression facing the distal end of the needle and abutting a space between adjacent depressions nearly perpendicular to the longitudinal axis A of the needle, and wherein the semi-spherical shape of the first reflector surface continues to the bottom of the depression and further in the proximal direction where the first surface transitions to the second reflector surface being a shallow portion extending to a second end portion of the depression at an angle less steep than that of the steep portion. And more specifically, the surface of the steep portion at the first end portion is close to a plane perpendicular to the longitudinal axis A of the needle sloping slightly toward a plane parallel to the longitudinal axis A, such that at least a portion of ultrasound waves from said transducer positioned anywhere in the range of slightly more than 0° to close to 90°, relative to the longitudinal axis A of the needle will impact a portion of the depression which is substantially perpendicular to a front of the wave sending the wave directly back to the transducer.

According to a further embodiment the steep portion is positioned to reflect waves back to a transducer aimed nearly parallel (close to 0°) to the needle 100 while the shallow portion, i.e. the second transducer surface (18), is oriented to reflect waves back to a transducer (10) positioned substantially perpendicular to the longitudinal axis A (close to 90°) of the needle.

This advantageous as the medical needle will reflect ultrasonic waves from a wide range of angles.

According to another embodiment the number of depressions of one depression section is five, six, seven or eight. This is advantageous as it also improves the ability to reflect ultrasonic waves as more depressions are arranged to face the direction of the ultrasonic waves.

According to still another embodiment the maximal width of a depression (8) in a plane perpendicular to the longitudinal axis A, is essentially equal to the minimum space between two depressions.

According to another embodiment straight radial lines through the bottoms of adjacent depressions in a cross-sectional plane are separated by a separation angle β, and that adjacent depression sections are off-set in relation to each other by an off-set angle being half the separation angle β. It will thereby be possible to increase the number of depressions, which in turn improves the ability to reflect ultrasonic waves.

In a still further embodiment adjacent depression sections are off-set in relation to each other by an off-set angle, and wherein the most distal parts of depressions in a first depression section being interleaved in relation to the most proximal parts of depressions in an adjacent second depression section located distally said first depression section. And according to another embodiment, the most distal parts of the depressions in a first depression section are located distally the most proximal parts of depressions in a second depression section by a predetermined distance d along the longitudinal axis A, and wherein said second depression section is adjacent and located distally said first depression section. These embodiments are advantageous as they includes features required to improve the number of depressions provided at the needle.

### Brief description of the drawings

Figure 1 is a simplified illustration showing a cross-sectional view of a transducer provided with two medical needles (for illustrative purposes) according to the present invention.
Figure 2 is a partly cross-sectional view illustrating detail A in figure 1.
Figure 3 is a partly cross-sectional view illustrating detail B in figure 1.
Figure 4 is a cross-sectional view of section C-C in figure 2.
Figure 5 is a cross-sectional view of section D-D in figure 2.
Figure 6 is a side-view of the medical needle according to the present invention.
Figure 7 is a side-view of detail A in figure 6.
Figure 8 is a cross-sectional view of section A-A in figure 7.
Figure 9 is a cross-sectional side view of section B-B in figure 7.
Figure 10 is a cross-sectional view of section C-C in figure 7.
Figure 11 is a perspective view of a part of the medical needle according to the present invention.
Figure 12 is a simplified illustration of a side view of a depression according to the present invention.

### Detailed description

The medical needle will now be described in detail with references to the appended figures. Throughout the figures the same, or similar, items have the same reference signs. Moreover, the items and the figures are not necessarily to scale, emphasis instead being placed upon illustrating the principles of the invention.

The present invention, which may be further understood with reference to the following description and the appended drawings, relates to medical needles e.g. for conducting biopsies under ultrasound guidance. Exemplary embodiments of the invention are directed to depressions on an outer surface of a needle such that the needle has enhanced ultrasound visibility, allowing the needle to remain visible at various angles relative to the transducer. It will be understood by those of skill in the art that although the exemplary embodiments are described as a needle, the device may be any medical device that may be seen under ultrasound guidance. It will also be understood by those of skill in the art that the medical needle provided with depressions as described herein, which enhance visibility, may also be used with other energy sources such as, for example, light.

As would be understood by those skilled in the art, the needle may be formed of any biocompatible material rigid enough to penetrate the tissue targeted by the procedure to which the needle is directed. For example, the needle may be formed of stainless steel or tungsten to enhance the echogenicity of the needle. As would be understood by those skilled in the art, tungsten has an acoustic impedance greater than that of stainless steel increasing the difference in acoustic impedance between the needle and the surrounding tissue and thereby enhancing echogenicity. It will be understood in the art, however, that any of a variety of materials may be used to form the needle so long as the material is biocompatible and provides a visible difference in echogenicity as compared to the tissue through which it will be deployed.

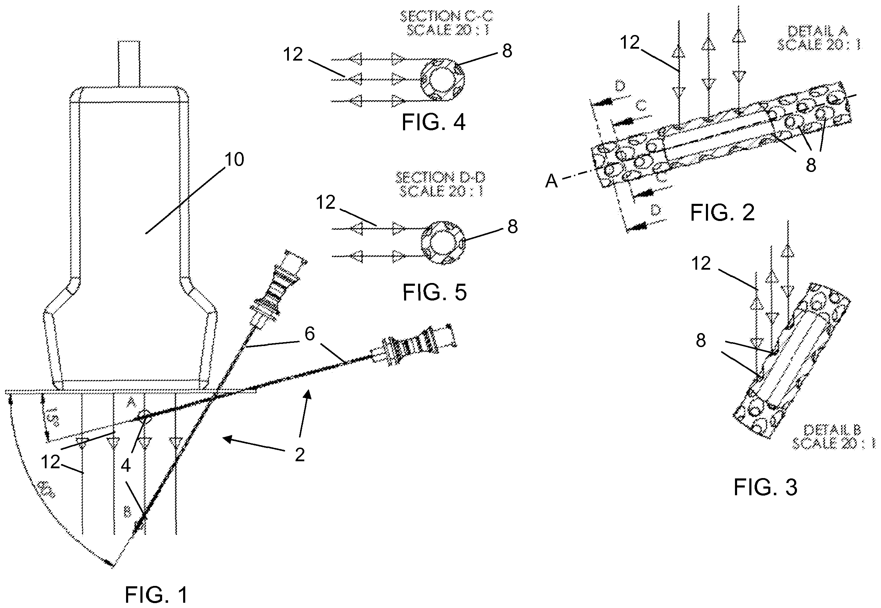

The medical needle will generally comprise a lumen extending there through to an opening in a distal tip at a distal end of the needle for collecting target tissue as would be understood by those skilled in the art. As shown in figure 1, the tip may be formed by a cut through the needle at an angle relative to a longitudinal axis of the longitudinal body so that a distal-most surface of the needle extends along an angle relative to a longitudinal axis of the needle with an area of the opening to the lumen greater than a cross-sectional area of the lumen within the needle.

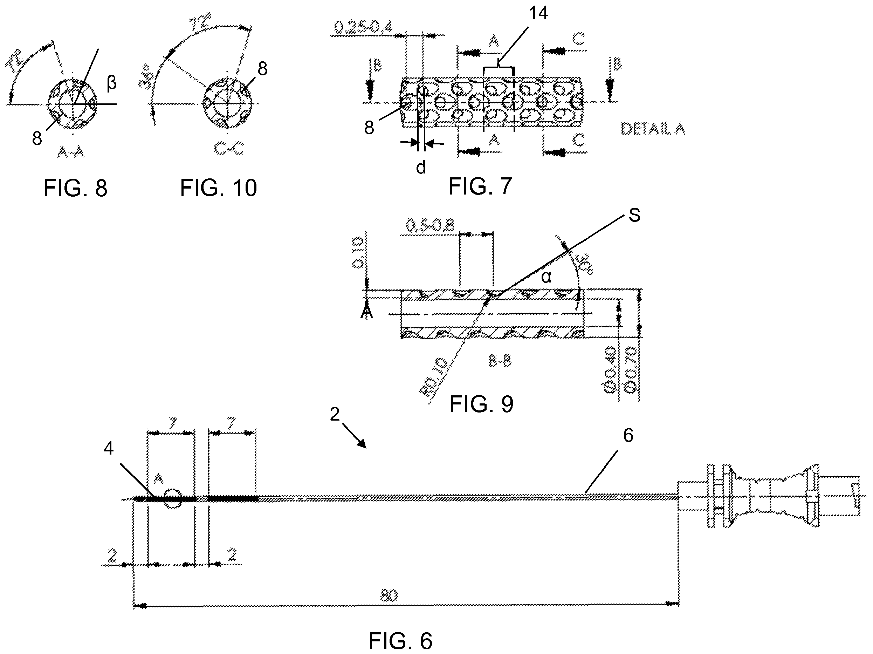

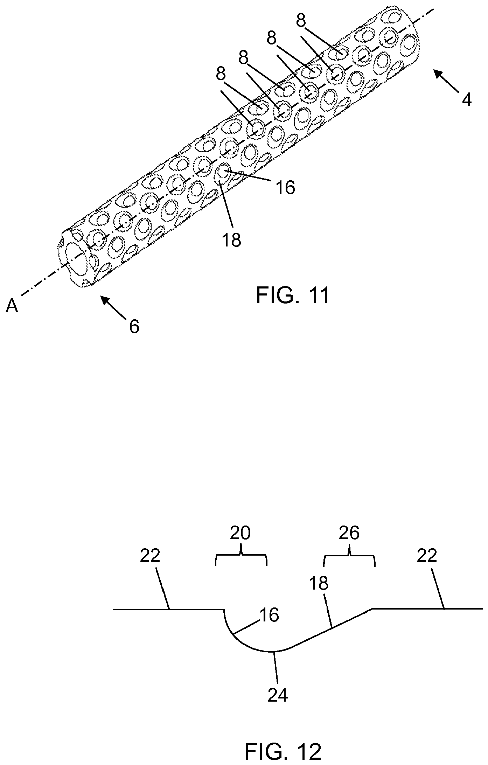

First with references to figure 1, the present invention relates to a medical needle 2 comprising a longitudinal body extending between a distal end 4 and a proximal end 6 along a longitudinal axis A (see figure 2). An outer surface of the needle 2 includes a plurality of concave echogenic ultrasonic reflector depressions 8 formed along at least a portion of a length of the needle to enhance the visibility of the needle under ultrasound guidance by scattering and reflecting back toward a transducer 10 sound waves 12 incident thereon. One row of the depressions 8 are evenly distributed around the entire circumference of a circular cylindrical section of the needle. The circular cylindrical section having depressions around the entire circumference is denoted a depression section 14 and is indicated in figure 7. Naturally, numerous depression sections are provided adjacent to each other. Each of the depressions 8 comprises two non-planar reflector surfaces, a first reflector surface 16 and a second reflector surface 18, see figures 11 and 12. The first reflector surface 16 is essentially a semi-spherical surface, and the second reflector surface 18 is a part of a cylindrical or a conical surface, and that the second reflector surface has a longitudinal symmetry axis S that is inclined in the direction of the proximal end 6 of the needle in relation to the longitudinal axis A of the needle by an inclination angle α being in the interval of 20 - 35 degrees. This is illustrated in figure 9.

More in detail, the two non-planar reflector surfaces may be achieved by first making a depression shaped as the semi-spherical surface. According to one variation the second reflector surface is a part of a cylindrical surface. The radius of the circular cylinder making up this surface may be essentially the same, or slightly larger than the radius of the sphere making up the first reflector surface. According to another variation the second reflector surface is a part of a conical surface. In particular the depression is made by using the surface of a right circular cone having a small apex angle. Also here, the radius of the part of the cone making up the second reflector surface is essentially the same, or slightly larger than the radius of the sphere making up the first reflector surface.

According to an embodiment which schematically is illustrated by figure 12, the first surface 16 of the depression 8 ranges from a steep portion extending from a first end portion 20 of the depression facing the distal end 4 of the needle 2 and abutting a space 22 between adjacent depressions 8 nearly perpendicular to the longitudinal axis A of the needle. The semi-spherical shape of the first reflector surface 16 continues to the bottom 24 of the depression 8 and further in the proximal direction where the first surface transitions to the second reflector surface 18 being a shallow portion extending to a second end portion 26 of the depression 8 at an angle less steep than that of the steep portion.

Preferably, the surface of the steep portion at the first end portion 20 is close to a plane perpendicular to the longitudinal axis A of the needle, sloping slightly toward a plane parallel to the longitudinal axis A, such that at least a portion of ultrasound waves from the transducer 10 positioned anywhere in the range of slightly more than 0° to close to 90°, relative to the longitudinal axis A of the needle will impact a portion of the depression 8 which is substantially perpendicular to a front of the wave sending the wave directly back to the transducer 10.

More particularly, the steep portion is positioned to reflect waves back to a transducer 10 aimed nearly parallel (close to 0°) to the needle 100 while the shallow portion, i.e. the second transducer surface 18, is oriented to reflect waves back to a transducer 10 positioned substantially perpendicular to the longitudinal axis A (close to 90°) of the needle.

The bottom 24 of a depression is defined as the position of the maximal depth of the depression, and that the bottom is closer to the first end than to the second end of the depression, along the longitudinal axis A.

Preferably, the number of depressions 8 on one depression section 14 around the needle is five, six, seven or eight.

According to an embodiment, which is illustrated by figure 8, straight radial lines through the bottoms 24 of adjacent depressions in a cross-sectional plane are separated by a separation angle β.

In the illustrated examples the number of depressions of one depression section is five. If a line is drawn through the bottom of each depression in a cross-sectional plane, i.e. being the radius of the circular cross-section of the needle, they are separated by a separation angle of 72 degrees to the adjacent depressions. This is illustrated in figures 8 and 10 which illustrate cross-sectional views along A-A and C-C, respectively, in figure 7.

According to another embodiment, also illustrated by figures 8 and 10, the maximal width of a depression 8 in a plane perpendicular to the longitudinal axis A, is essentially equal to the minimum space between two depressions.

According to still another embodiment adjacent depression sections 14 are off-set in relation to each other by an off-set angle being half the separation angle β. If the separation angle is 72 degrees the off-set angle is then 36 degrees. By arranging adjacent depression sections off-set in relation to each other it is possible to increase the number of depressions on a predetermined surface area. Furthermore, adjacent depression sections 14 are off-set in relation to each other by an off-set angle, and the most distal parts of depressions in a first depression section being interleaved in relation to the most proximal parts of depressions in an adjacent second depression section located distally the first depression section. This is illustrated in figure 7.

In a further embodiment, the most distal parts of the depressions in a first depression section are located distally the most proximal parts of depressions in a second depression section by a predetermined distance d (see figure 7) along the longitudinal axis A, and wherein the second depression section is adjacent and located distally the first depression section.

One object of the second reflector surface is to open up the first reflector surface in the proximal direction of each depression. Thereby the visibility of the needle is improved for ultrasound waves along a direction having an angle in relation to the longitudinal axis A that is small, i.e. between 0 and 40 degrees.

An important aspect achieved by the first surface having an essentially semi-spherical shape is that the first surface also will reflect ultrasound waves even if the depression not is directly facing the direction of the incoming waves - this is illustrated by figure B-B.

More particularly, the depressions, as shown in the enlarged side view of figures 2 and 3, are shaped to directly reflect sound waves received over a broad range of angles so that the transducer may be placed in a variety of positions relative to the needle. Figure 2 illustrates detail A of the needle in figure 1 where the incoming angles of the sound waves is close to 90 degrees, whereas figure 3 illustrates detail B of the needle in figure 1 where the incoming angles of the sound waves are more acute in relation to the longitudinal axis A.

That is, the shapes of the depressions are selected to present at least a part of a face thereof substantially perpendicular to incoming ultrasound radiation over a wide range of incoming angles so that this radiation will be reflected back to the device from which it originated.

In figures 6-10 some examples of dimensions of various parts of the needle are included. These are purely included as examples, and do not exclude other dimensions.

The needle illustrated in figure 6 is provided with two portions of depressions at its distal end. Each portion has a length of 7 mm, and are separated by a 2 mm gap.

The distance between the same parts (e.g. bottoms) of depressions in adjacent depression sections is in the interval of 0.25 - 0.4 mm (see figure 7).

The longitudinal distance between the same parts (e.g. bottoms) of depressions along a line parallel to axis A, i.e. in depression sections having the same orientation with one depression section in-between, is in the interval of 0. 5 - 0.8 mm (see figure 7).

Also illustrated in figure 7 is the depth of a depression which is 0.10 mm. The radius of the semi-spherical first reflector surface is e.g. 0.10 mm. The outer diameter of the needle is 0.70 mm, whereas the inner diameter is 0.40 mm.

It will be understood by those of skill in the art that the features of the needle 2, as described above may also be included in other medical devices that may be viewed under ultrasound guidance. For example, a sheath, which may be slidable along a portion of a length of a needle may include a pattern substantially similar to the pattern formed by the depressions 8 on the needle 2. In another variation, a stylet, which may be slidable through a lumen of a needle to prevent non-target tissue from entering the lumen may be formed with a pattern substantially similar to the pattern formed by the depressions 8 on the needle 2.

The present invention is not limited to the above-described preferred embodiments. Various alternatives and modifications may be used.

Therefore, the above embodiments should not be taken as limiting the scope of the invention, which is defined by the appending claims.

## Claims

1. A medical needle (2) comprising a longitudinal body extending between a distal end (4) and a proximal end (6) along a longitudinal axis A, an outer surface of the needle (2) includes a plurality of concave echogenic ultrasonic reflector depressions (8) formed along at least a portion of a length of the needle to enhance the visibility of the needle under ultrasound guidance by scattering and reflecting back toward a transducer (10) sound waves (12) incident thereon, a row of said depressions (8) are evenly distributed around the entire circumference of a circular cylindrical section of the needle, said circular cylindrical section having depressions around the entire circumference is a depression section (14), **characterized in that** each of said plurality of depressions (8) comprises two non-planar reflector surfaces, a first reflector surface (16) and a second reflector surface (18), wherein the first reflector surface (16) is a semi-spherical surface, and the second reflector surface (18) is a part of a cylindrical or a conical surface, wherein the second reflector surface having a longitudinal symmetry axis S that is inclined in the direction of the proximal end (6) of the needle in relation to the longitudinal axis A of the needle by an inclination angle α being in the interval of 20 - 35 degrees.

2. The medical needle (2) according to claim 1, wherein the first surface (16) of the depression (8) ranges from a steep portion extending from a first end portion (20) of the depression facing the distal end (4) of the needle (2) and abutting a space (22) between adjacent depressions (8) nearly perpendicular to the longitudinal axis A of the needle, and wherein the semi-spherical shape of the first reflector surface (16) continues to the bottom (24) of the depression (8) and further in the proximal direction where the first surface transitions to the second reflector surface (18) being a shallow portion extending to a second end portion (26) of the depression (8) at an angle less steep than that of the steep portion.

3. The medical needle (2) according to claim 2, wherein the surface of the steep portion at said first end portion (20) is close to a plane perpendicular to the longitudinal axis A of the needle sloping slightly toward a plane parallel to the longitudinal axis A, such that at least a portion of ultrasound waves from said transducer (10) positioned anywhere in the range of slightly more than 0° to close to 90°, relative to the longitudinal axis A of the needle will impact a portion of the depression (8) which is substantially perpendicular to a front of the wave sending the wave directly back to the transducer (10).

4. The medical needle (2) according to claim 2 or 3, wherein said steep portion is positioned to reflect waves back to a transducer (10) aimed nearly parallel (close to 0°) to the needle 100 while the shallow portion, i.e. the second transducer surface (18), is oriented to reflect waves back to a transducer (10) positioned substantially perpendicular to the longitudinal axis A (close to 90°) of the needle.

5. The medical needle (2) according to any of claims 1-4, wherein the number of depressions (8) of one depression section (14) is five, six, seven or eight.

6. The medical needle (2) according to any of claims 1-5, wherein the maximal width of a depression (8) in a plane perpendicular to the longitudinal axis A, is essentially equal to the minimum space between two depressions.

7. The medical needle (2) according to any of claims 1-6, wherein straight radial lines through bottoms (24) of adjacent depressions in a cross-sectional plane are separated by a separation angle β.

8. The medical needle (2) according to claim 7, wherein adjacent depression sections (14) are off-set in relation to each other by an off-set angle being half the separation angle β.

9. The medical needle (2) according to any of claims 1-8, wherein adjacent depression sections (14) are off-set in relation to each other by an off-set angle, and wherein the most distal parts of depressions in a first depression section being interleaved in relation to the most proximal parts of depressions in an adjacent second depression section located distally said first depression section.

10. The medical needle (2) according to any of claims 1-9, wherein the most distal parts of the depressions in a first depression section are located distally the most proximal parts of depressions in a second depression section by a predetermined distance d along the longitudinal axis A, and wherein said second depression section is adjacent and located distally said first depression section.

## Patentansprüche

1. Medizinische Nadel (2), umfassend einen Längskörper, der sich zwischen einem distalen Ende (4) und einem proximalen Ende (6) entlang einer Längsachse A erstreckt, wobei eine äußere Oberfläche der Nadel (2) eine Vielzahl von konkaven echogenen Ultraschallreflektorvertiefungen (8) enthält, die entlang mindestens eines Teils einer Länge der Nadel ausgebildet sind, um die Sichtbarkeit der Nadel unter Ultraschallführung zu verbessern, indem darauf einfallende Schallwellen (12) gestreut und zu einem Wandler (10) zurückreflektiert werden, wobei eine Reihe der Vertiefungen (8) gleichmäßig um den gesamten Umfang eines kreiszylindrischen Abschnitts der Nadel verteilt ist, wobei der kreiszylindrische Abschnitt mit Vertiefungen um den gesamten Umfang ein Vertiefungsabschnitt (14) ist, **dadurch gekennzeichnet, dass** jede der Vielzahl von Vertiefungen (8) zwei nicht ebene Reflektorflächen, eine erste Reflektorfläche (16) und eine zweite Reflektorfläche (18), umfasst, wobei die erste Reflektorfläche (16) eine halbkugelförmige Fläche ist und die zweite Reflektorfläche (18) ein Teil einer zylindrischen oder konischen Fläche ist, wobei die zweite Reflektorfläche eine Längssymmetrieachse S aufweist, die in Richtung des proximalen Endes (6) der Nadel in Bezug auf die Längsachse A der Nadel um einen Neigungswinkel α geneigt ist, der im Bereich von 20 bis 35 Grad liegt.

2. Medizinische Nadel (2) nach Anspruch 1, wobei sich die erste Fläche (16) der Vertiefung (8) von einem steilen Abschnitt erstreckt, der sich von einem ersten Endabschnitt (20) der Vertiefung erstreckt, der dem distalen Ende (4) der Nadel (2) zugewandt ist und an einen Raum (22) zwischen benachbarten Vertiefungen (8) nahezu senkrecht zur Längsachse A der Nadel angrenzt, und wobei sich die halbkugelförmige Form der ersten Reflektorfläche (16) zum Boden (24) der Vertiefung (8) und weiter in die proximale Richtung fortsetzt, wo die erste Fläche in die zweite Reflektorfläche (18) übergeht, die ein flacher Abschnitt ist, der sich zu einem zweiten Endabschnitt (26) der Vertiefung (8) in einem Winkel erstreckt, der weniger steil ist als der des steilen Abschnitts.

3. Medizinische Nadel (2) nach Anspruch 2, wobei die Fläche des steilen Abschnitts an dem ersten Endabschnitt (20) nahe einer Ebene senkrecht zur Längsachse A der Nadel liegt und leicht zu einer Ebene parallel zur Längsachse A geneigt ist, so dass zumindest ein Teil der Ultraschallwellen von dem Wandler (10), der irgendwo im Bereich von etwas mehr als 0° bis nahezu 90° relativ zur Längsachse A der Nadel positioniert ist, auf einen Teil der Vertiefung (8) auftrifft, der im Wesentlichen senkrecht zu einer Front der Welle ist und die Welle direkt zum Wandler (10) zurückschickt.

4. Medizinische Nadel (2) nach Anspruch 2 oder 3, wobei der steile Abschnitt so angeordnet ist, dass er Wellen zu einem Wandler (10) zurückreflektiert, der nahezu parallel (nahe 0°) zur Nadel 100 ausgerichtet ist, während der flache Abschnitt, d.h. die zweite Wandlerfläche (18), so ausgerichtet ist, dass er Wellen zu einem Wandler (10) zurückreflektiert, der im Wesentlichen senkrecht zur Längsachse A (nahe 90°) der Nadel angeordnet ist.

5. Medizinische Nadel (2) nach einem der Ansprüche 1 bis 4, wobei die Anzahl der Vertiefungen (8) eines Vertiefungsabschnitts (14) fünf, sechs, sieben oder acht beträgt.

6. Medizinische Nadel (2) nach einem der Ansprüche 1 bis 5, wobei die maximale Breite einer Vertiefung (8) in einer Ebene senkrecht zur Längsachse A im Wesentlichen gleich dem Mindestabstand zwischen zwei Vertiefungen ist.

7. Medizinische Nadel (2) nach einem der Ansprüche 1 bis 6, wobei gerade radiale Linien durch Böden (24) benachbarter Vertiefungen in einer Querschnittsebene durch einen Trennwinkel β getrennt sind.

8. Medizinische Nadel (2) nach Anspruch 7, wobei benachbarte Vertiefungsabschnitte (14) in Bezug aufeinander um einen Versatzwinkel versetzt sind, der die Hälfte des Trennwinkels β beträgt.

9. Medizinische Nadel (2) nach einem der Ansprüche 1 bis 8, wobei benachbarte Vertiefungsabschnitte (14) in Bezug aufeinander um einen Versatzwinkel versetzt sind und wobei die distalsten Teile von Vertiefungen in einem ersten Vertiefungsabschnitt in Bezug auf die proximalsten Teile von Vertiefungen in einem benachbarten zweiten Vertiefungsabschnitt, der distal von dem ersten Vertiefungsabschnitt angeordnet ist, verschachtelt sind.

10. Medizinische Nadel (2) nach einem der Ansprüche 1 bis 9, wobei die distalsten Teile der Vertiefungen in einem ersten Vertiefungsabschnitt um einen vorbestimmten Abstand d entlang der Längsachse A distal von den proximalsten Teilen der Vertiefungen in einem zweiten Vertiefungsabschnitt angeordnet sind, und wobei der zweite Vertiefungsabschnitt an den ersten Vertiefungsabschnitt angrenzt und distal von diesem angeordnet ist.

## Revendications

1. Aiguille médicale (2) comprenant un corps longitudinal s'étendant entre une extrémité distale (4) et une extrémité proximale (6) le long d'un axe longitudinal A, une surface extérieure de l'aiguille (2) inclut une pluralité de creux de réflecteur ultrasonore échogènes concaves (8) formés le long d'au moins une partie d'une longueur de l'aiguille pour améliorer la visibilité de l'aiguille sous guidage ultrasonore par diffusion et réflexion en retour vers un transducteur (10) des ondes sonores (12) incidentes sur celle-ci, une rangée desdits creux (8) sont répartis uniformément sur toute la circonférence d'une section cylindrique circulaire de l'aiguille, ladite section cylindrique circulaire présentant des creux sur toute la circonférence est une section de creux (14), **caractérisée en ce que** chacun de ladite pluralité de creux (8) comprend deux surfaces de réflecteur non planes, une première surface de réflecteur (16) et une seconde surface de réflecteur (18), dans laquelle la première surface de réflecteur (16) est une surface semi-sphérique, et la seconde surface de réflecteur (18) est une partie d'une surface cylindrique ou conique, dans laquelle la seconde surface de réflecteur présente un axe de symétrie longitudinal S qui est incliné dans la direction de l'extrémité proximale (6) de l'aiguille par rapport à l'axe longitudinal A de l'aiguille par un angle d'inclinaison α étant dans l'la plage de 20 à 35 degrés.

2. Aiguille médicale (2) selon la revendication 1, dans laquelle la première surface (16) du creux (8) s'étend à partir d'une partie abrupte s'étendant à partir d'une première partie d'extrémité (20) du creux faisant face à l'extrémité distale (4) de l'aiguille (2) et venant en butée contre un espace (22) entre des creux adjacents (8) presque perpendiculaire à l'axe longitudinal A de l'aiguille, et dans laquelle la forme semi-sphérique de la première surface de réflecteur (16) se poursuit jusqu'au fond (24) du creux (8) et en outre dans la direction proximale où la première surface effectue une transition vers la seconde surface de réflecteur (18) étant une partie peu profonde s'étendant jusqu'à une seconde partie d'extrémité (26) du creux (8) selon un angle moins abrupt que celui de la partie abrupte.

3. Aiguille médicale (2) selon la revendication 2, dans laquelle la surface de la partie abrupte au niveau de ladite première partie d'extrémité (20) est proche d'un plan perpendiculaire à l'axe longitudinal A de l'aiguille incliné légèrement vers un plan parallèle à l'axe longitudinal A, de telle sorte qu'au moins une partie des ondes ultrasonores provenant dudit transducteur (10) positionnée n'importe où dans la plage allant d'un peu plus de 0° à près de 90°, par rapport à l'axe longitudinal A de l'aiguille, va heurter une partie du creux (8) qui est sensiblement perpendiculaire à un front de l'onde en renvoyant l'onde directement vers le transducteur (10).

4. Aiguille médicale (2) selon la revendication 2 ou 3, dans laquelle ladite partie abrupte est positionnée de manière à réfléchir les ondes en retour vers un transducteur (10) orienté presque parallèlement (presque à 0°) à l'aiguille 100 tandis que la partie peu profonde, c'est-à-dire la seconde surface de transducteur (18), est orientée de manière à réfléchir les ondes en retour vers un transducteur (10) positionné sensiblement perpendiculairement à l'axe longitudinal A (presqu'à 90°) de l'aiguille.

5. Aiguille médicale (2) selon l'une quelconque des revendications 1 à 4, dans laquelle le nombre de creux (8) d'une section de creux (14) est de cinq, six, sept ou huit.

6. Aiguille médicale (2) selon l'une quelconque des revendications 1 à 5, dans laquelle la largeur maximale d'un creux (8) dans un plan perpendiculaire à l'axe longitudinal A, est essentiellement égale à l'espace minimum entre deux creux.

7. Aiguille médicale (2) selon l'une quelconque des revendications 1 à 6, dans laquelle des lignes radiales droites à travers des fonds (24) de creux adjacents dans un plan de section transversale sont séparées par un angle de séparation β.

8. Aiguille médicale (2) selon la revendication 7, dans laquelle des sections de creux adjacentes (14) sont décalées les unes par rapport aux autres d'un angle de décalage correspondant à la moitié de l'angle de séparation β.

9. Aiguille médicale (2) selon l'une quelconque des revendications 1 à 8, dans laquelle des sections de creux adjacentes (14) sont mutuellement décalées d'un angle de décalage, et dans laquelle les parties les plus distales des creux dans une première section de creux sont entremêlées par rapport aux parties les plus proximales des creux dans une seconde section de creux adjacente située manière distale par rapport à ladite première section de creux.

10. Aiguille médicale (2) selon l'une quelconque des revendications 1 à 9, dans laquelle les parties les plus distales des creux dans une première section de creux sont situées de manière distale par rapport aux parties les plus proximales de creux dans une seconde section de creux d'une distance prédéterminée d le long de l'axe longitudinal A, et dans laquelle ladite seconde section de creux est adjacente et située manière distale par rapport à ladite première section de creux.
